# EUROPEAN PATENT SPECIFICATION

(11) **EP 3 965 752 B1**
(45) Date of publication and mention of the grant of the patent: **30.08.2023**
(21) Application number: 20727843.3
(22) Date of filing: 06.05.2020
(51) Int. Cl.: A61K 31/195, A61K 31/496, A61P 25/02, A61P 25/04, A61K 45/06

(54) **PHARMACEUTICAL COMBINATION COMPRISING TRAZODONE FOR THE TREATMENT OF NEUROPATHIC PAIN**
PHARMAZEUTISCHE KOMBINATION MIT TRAZODON ZUR BEHANDLUNG VON NEUROPATHISCHEN SCHMERZEN
COMBINAISON PHARMACEUTIQUE COMPRENANT DE LA TRAZODONE POUR LE TRAITEMENT DE LA DOULEUR NEUROPATHIQUE

(30) Priority: 07.05.2019 IT 201900006602
(43) Date of publication of application: 16.03.2022
(73) Proprietor: Aziende Chimiche Riunite Angelini Francesco A.C.R.A.F. S.p.A., 00181 Roma (IT)
(72) Inventor: POLENZANI, Lorenzo, 00046 Grottaferrata (RM) (IT); CALISTI, Fabrizio, 00142 Rome (IT); LIPONE, Paola, 04100 Latina (LT) (IT); TONGIANI, Serena, 00046 Grottaferrata (RM) (IT)
(74) Representative: PGA S.p.A.
(86) International application number: PCT/IB2020/054275
(87) International publication number: WO 2020/225740

(56) References cited:
- WO-A1-2017/067870
- WO-A2-2009/021058
- US-A1- 2008 096 872
- US-A1- 2012 083 508
- WILSON RC1: "The use of low-dose trazodone in the treatment of painful diabetic neuropathy", JOURNAL OF THE AMERICAN PODIATRIC MEDICAL ASSOCIATION, AMERICAN PODIATRIC MEDICAL ASSOCIATION, USA, vol. 89, no. 9, 1 September 1999 (1999-09-01), pages 468-471, XP009189323, ISSN: 8750-7315, DOI: 10.7547/87507315-89-9-468)
- OKUDA K1 ET AL: "Trazodone hydrochloride attenuates thermal hyperalgesia in a chronic constriction injury rat model", EUROPEAN JOURNAL OF ANAESTHESIOLOGY, PUBL. ON BEHALF OF THE EUROPEAN ACADEMY OF ANAESTHESIOLOGY AND THE EUROPEAN SOCIETY OF ANAESTHESIOLOGISTS, GB, vol. 20, no. 5, 1 May 2003 (2003-05-01), pages 409-415, XP009189324, ISSN: 0265-0215, DOI: 10.1017/S0265021503000632

## Description

### FIELD OF INVENTION

The present invention relates to a pharmaceutical composition consisting of a combination of trazodone and gabapentin or pregabalin or mirogabalin with an improving effect in the treatment of neuropathic pain, in particular pain resulting from diabetic neuropathy.

### STATE OF THE ART

Physiological pain is an important protective mechanism designed to warn of danger from potentially harmful stimuli from the external environment. Neuropathic pain is defined as pain initiated or caused by a primary injury or dysfunction of the nervous system. Neuropathic pain is often debilitating and causes loss of ability to work and poor quality of life. It also leads to economic and social harm.

Analgesic drugs currently used in the treatment of neuropathic pain include nonsteroidal anti-inflammatory drugs (NSAIDs), antidepressants, opiate analgesics and anticonvulsants [Woolf CJ, Mannion RJ, Neuropathic pain: aetiology, symptoms, mechanisms, and management. Lancet 1999; 353: 1959-1964].

However, neuropathic pain is notoriously difficult to treat with currently available medications. Consequently, the development of new drugs has always been one of the main objectives of the pharmaceutical industry.

Diabetic neuropathy is one of the most common long-term symptomatic complications in patients with both type 1 and type 2 diabetes mellitus. At initial diagnosis, 7.5% of patients already suffer from painful diabetic peripheral neuropathy and about 45-50% will suffer from this complication after 25 years.

Diabetic neuropathic pain is generally described as tingling, burning, stinging, stabbing, or even an electric shock sensation. It is usually considered moderate to severe and often worsens at night causing sleep disturbances. Pain can be constant and accompanied by skin allodynia, which can substantially affect patients' quality of life, affecting their ability to perform daily activities and having a negative influence on their mood. Pain can also be a reason for withdrawal from recreational and social activities and can be associated with depression.

Although traditionally the first step in diabetic neuropathic pain management has been to improve and optimize glycemic control, additional pain control medications are usually needed.

Current treatment options include antidepressants such as tricyclic agents and serotonin-norepinephrine reuptake inhibitors, γ-aminobutyric acid (GABA) analogues such as pregabalin, gabapentin and mirogabalin, opioids and topical treatments.

Gabapentin is a known anticonvulsant, currently approved for the treatment of epilepsy and neuropathic pain. Gabapentin is effective in neuropathic pain management and management of diabetic neuropathy has found in gabapentin the best balance between safety and efficacy.

Gabapentin is now widely recommended as a first-line treatment for peripheral diabetic neuropathy by several guidelines such as the American Academy of Neurology, National Institute for Health and Care Excellence, European Federation of Neurological Societies and Neuropathic Pain Special Interest Group of the International Association for the Study of Pain.

Unfortunately, there is no consensus on a single more effective drug and monotherapy rarely provides adequate pain relief. Clinical management of diabetic neuropathic pain continues to pose a therapeutic challenge and the response to existing treatments is often inadequate. A wide range of drugs, used alone or in combination, have been shown to significantly reduce neuropathic pain compared to placebo in randomized controlled trials, but pain relief remains inadequate for most patients.

Trazodone is a well-known antidepressant with a multi-receptor mechanism of action and is proposed as an alternative treatment to approved pain medications as one of the potentially effective off-label drugs. Low doses of trazodone have already been used in the treatment of neuropathic pain.

Khurana observed a good effect of trazodone in pain relief and numbness in 6 diabetic patients suffering from peripheral diabetic neuropathy when administered at 100 mg/day in a divided dose (Khurana RC., "Treatment of painful diabetic neuropathy with trazodone, JAMA vol. 250, no. 11, 1983).

Subsequently, 31 adult diabetic patients with painful distal symmetrical polyneuropathy were treated with oral trazodone doses of 50 or 100 mg/day. After 2 weeks of therapy, 61.3% of patients had symptomatic relief and 22.6% had complete relief. After the end of the study all patients who had obtained symptomatic relief continued to take trazodone without reduction of results or development of side effects. The authors concluded that trazodone appeared to be a good alternative to tricyclic antidepressants considering its efficacy, safety profile and rapid onset of action (Wilson RC., "The use of low dose Trazodone in the treatment of painful diabetic neuropathy", J. Am. Podiatr. Med. Assoc. 1999 Sep; 89(9):468-71).

EP1663398B1 describes a combination of an alpha-2-delta ligand such as gabapentin and pregabalin with a selective noradrenaline reuptake inhibitor such as (S,S)-reboxetine for the treatment of neuropathic pain.

WO2013/002584 describes a pain relief composition that comprises two or more components selected from (a) a 5-HT2 receptor antagonist, (b) a P2X receptor antagonist, and (c) any of the glycine receptor agonists, a glycine transporter antagonist, a gamma-aminobutyric acid receptor agonist (GABA), and a GABA 1 transporter antagonist (GABA1).

WO2017/067870 describes a pharmaceutical composition comprising a synergistic combination of trazodone in an amount such as to provide a dose equal to or lower than 1 mg/kg and gabapentin in an amount such as to provide a dose equal to or lower than 15 mg/kg, with a weight ratio of trazodone and gabapentin between 1:15 and 1:5, for use in the treatment of chronic pain, in particular inflammatory pain induced in mice by acetic acid injection, and neuropathic pain induced in rats by sciatic nerve ligation.

A review of the use of gabapentin in the treatment of neuropathic pain was published by Kukkar A. et al, "Implications and mechanism of action of gabapentin in neuropathic pain", Arch. Pharm. Ris. (2013) 36:237-251. A superior effect has been observed by the combination of gabapentin and venlafaxine in the neuropathic pain model and the co-administration of gabapentin with donepezil and/or duloxetine against neuropathic pain induced by spinal nerve ligation and in spared nerve injury model.

Trazodone and a combination of trazodone and pregabalin have been studied in a 12- and 24-week treatment of fibromyalgia as disclosed in Morillas-Arques et al., "Trazodone for the treatment of fibromyalgia: an open-label, 12-week study", BMC Musculoskeletal Disorders 2010, 11:204 and Calandre et al., "Trazodone plus pregabalin combination in the treatment of fibromyalgia: a two-phase, 24-week, open-label uncontrolled study", BMC Musculoskeletal Disorders 2011, 12:95.

A combination of trazodone with fentanyl and paracetamol has been studied in a mouse model of visceral pain, showing a potent synergistic antinociceptive effect, as disclosed in Fernández-Dueñas et al., "Fentanyl-trazodone-paracetamol triple drug combination: Multimodal analgesia in a mouse model of visceral pain", Pharmacology, Biochemistry and Behavior 98 (2011) 331-336.

### SUMMARY OF THE INVENTION

The Applicant has faced the problem of providing a pharmaceutical composition for the treatment of neuropathic pain, in particular pain resulting from diabetic neuropathy, with better activity than the compositions known in art.

In view of the results known in the art, the Applicant hypothesized that the combination of trazodone and gabapentin could be used for the treatment of diabetic neuropathy pain, and neuropathic pain in general.

The Applicant has surprisingly found that trazodone can be used in combination with gabapentin in very low relative quantities, with a weight ratio between trazodone and gabapentin between 1:40 and 1:100, preferably lower than 1:40 and up to 1:100.

In particular, the Applicant has surprisingly found that the combination of trazodone and gabapentin with a weight ratio between 1:40 and 1 :100, preferably lower than 1:40 and up to 1:100 has an improving effect in reducing pain from diabetic neuropathy in subjects treated for nine weeks with incremental gabapentin therapy to which trazodone co-administration has been added.

The effect found by the Applicant is even more surprising as combinations with a weight ratio between trazodone and gabapentin greater than 1:40 (e.g. 1:30) showed almost no improvement in reducing diabetic neuropathy pain.

The Applicant also found a surprisingly positive effect in reducing the risk of depressive or anxiety disorders in the same subjects treated with the aforementioned combination of gabapentin and trazodone.

In view of the results obtained with gabapentin, the Applicant believes that a similar effect can also be found with pregabalin or mirogabalin, active ingredients similar to gabapentin with the same therapeutic effect and mechanism of action but with greater power, using them in association with trazodone with the same weight ratio.

Therefore, in a first aspect, the present invention relates to a pharmaceutical composition which comprises (a) a combination of (i) a first active ingredient selected from the group consisting of trazodone and a salt thereof, and (ii) a second active ingredient selected from the group consisting of gabapentin, pregabalin, mirogabalin, a salt thereof, and a prodrug thereof, and (b) at least one pharmaceutically acceptable excipient, for use in the treatment of pain resulting from diabetic neuropathy, wherein said pharmaceutical composition comprises a weight ratio of said first active ingredient to said second active ingredient, expressed as weight ratio of trazodone : gabapentin or pregabalin or mirogabalin, between 1:40 and 1 :100, preferably lower than 1:40 and up to 1:100.

In a second aspect, the present invention relates to a method for the treatment of pain resulting from diabetic neuropathy in a subject in need thereof that comprises the administration of an effective amount of a pharmaceutical composition that comprises (a) a combination of (i) a first active ingredient selected from the group consisting of trazodone and a salt thereof, and (ii) a second active ingredient selected from the group consisting of gabapentin, pregabalin, mirogabalin, a salt thereof, and a prodrug thereof, and (b) at least one pharmaceutically acceptable excipient, wherein said pharmaceutical composition comprises a weight ratio of said first active ingredient to said second active ingredient, expressed as weight ratio of trazodone : gabapentin or pregabalin or mirogabalin, between 1:40 and 1 :100, preferably lower than 1:40 and up to 1:100.

### BRIEF DESCRIPTION OF THE DRAWINGS

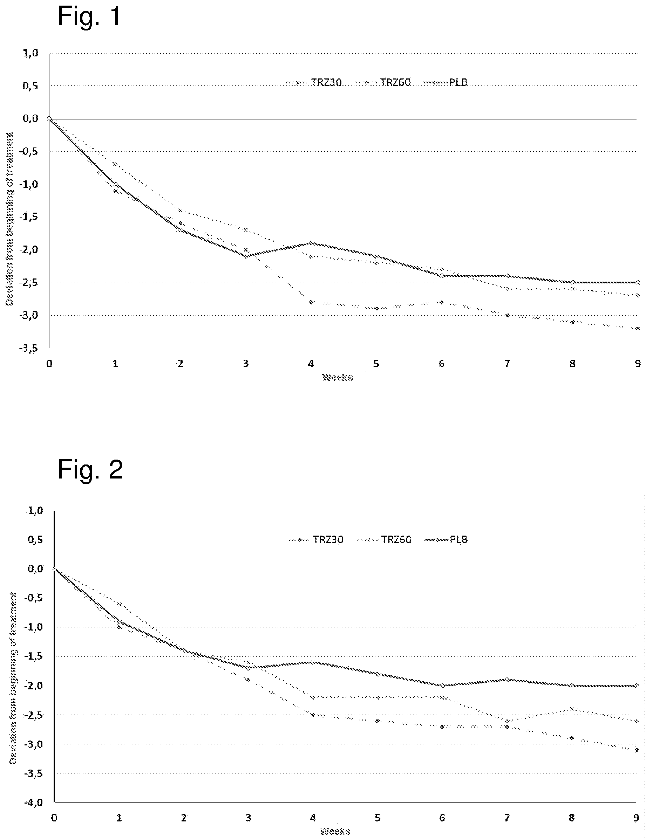

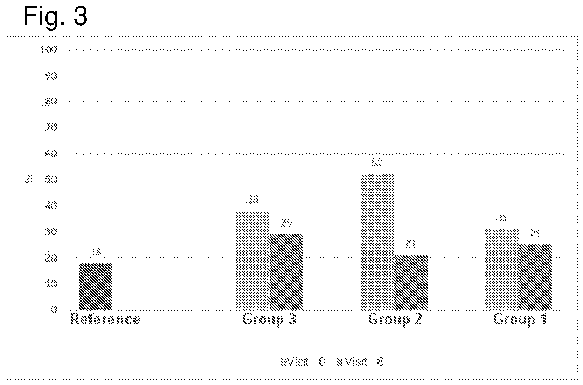

Figures 1 to 3 show graphically the results of the clinical study described in Example 1. In Figures 1 and 2, the mean deviations from the value at the beginning of treatment of pain perception by treated subjects are represented on the y-axis, while the abscissae axis shows the weeks of treatment. In Figure 3 the y-axis shows the percentage of patients with a positive risk of depression or anxiety in the normal population (Reference) and in the three groups treated (Group 1-3), at the beginning and at the end of treatment.

### DETAILED DESCRIPTION OF THE INVENTION

The expression "expressed as a weight ratio of trazodone : gabapentin or pregabalin or mirogabalin" as used in the present description and claims means that the ratio between the first active ingredient and the second active ingredient is always expressed between the equivalent weight of trazodone as such contained in the pharmaceutical composition (even if present as salt) and the equivalent weight of gabapentin or pregabalin or mirogabalin as such contained in the pharmaceutical composition (even if present as salt or prodrug).

The term 'trazodone' as used in the present description and claims represents the active ingredient 2-(3-[4-(3-chlorophenyl)piperazin-1-yl]propyl)-[1,2,4]triazole[4,3-a]pyridin-3(2H)-one having the following structural formula (I):

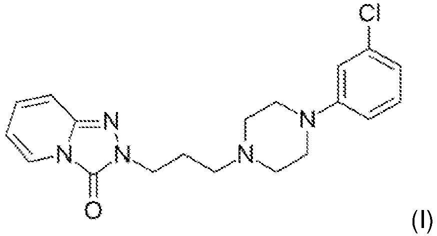

The term 'gabapentin' as used in the present description and claims represents the active ingredient 2-[1-(aminomethyl)cyclohexyl] acetic acid having the following structural formula (II):

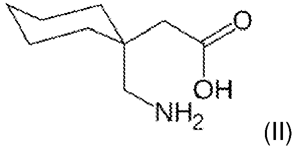

The term 'pregabalin' as used in the present description and claims represents the active ingredient (S)-3-(aminomethyl)-5-methylhexanoic acid having the following structural formula (III):

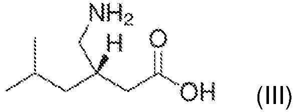

The term 'mirogabalin' as used in the present description and claims represents the active ingredient (1R,5S,6S)-6-(aminomethyl)-3-ethyl-bicycle(3.2.0)ept-3-ene-6-acetic acid having the following structural formula (IV):

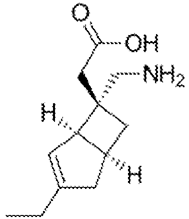

The pharmaceutical composition according to the present invention contains (i) a quantity of a first active ingredient selected from the group consisting of trazodone and a salt thereof, and (ii) a quantity of a second active ingredient selected from the group consisting of gabapentin, pregabalin, mirogabalin, a salt thereof, and a prodrug thereof, such as to provide a weight ratio of said first active ingredient and said second active ingredient, expressed as weight ratio of trazodone : gabapentin or pregabalin or mirogabalin, between 1:40 and 1:100, preferably lower than 1:40 and up to 1 :100, more preferably between 1:50 and 1:90, and even more preferably between 1:60 and 1:80.

According to a preferred aspect of the present invention, any value within the above mentioned trazodone : gabapentin or pregabalin or mirogabalin weight ratio can be used, such as, for example, 1:41, 1:42, 1:43, 1:44, 1:45, 1:46, 1:47, 1:48, 1:49, 1:50, 1:51, 1:52, 1:53, 1:54, 1:55, 1:56, 1:57, 1:58, 1:59, 1:60, 1:61, 1:62, 1:63, 1:64, 1:65, 1:66, 1:67, 1:68, 1:69, 1:70, 1:71, 1:72, 1:73, 1:74, 1:75, 1:76, 1:77, 1:78, 1:79, 1:80, 1:81, 1:82, 1:83, 1:84, 1:85, 1:86, 1:87, 1:88, 1:89, 1:90, 1:91, 1:92, 1:93, 1:94, 1:95, 1:96, 1:97, 1:98, 1:99, and 1:100.

The quantity of the first and second active ingredient contained in the pharmaceutical composition according to the present invention may be variable according to the dosage necessary to achieve the desired effect, provided that the weight ratio of said first active ingredient and said second active ingredient, expressed as weight ratio of trazodone : gabapentin or pregabalin or mirogabalin, remains between 1:40 and 1:100, preferably lower than 1:40 and up to 1:100, more preferably between 1:50 and 1:90, and even more preferably between 1:60 and 1:80.

Advantageously, the pharmaceutical composition of the present invention comprises a quantity of first active ingredient equivalent to a quantity of trazodone between 2.50 and 1.00 mg, preferably from 2.00 to 1.11 mg, and more preferably from 1.67 to 1.25 mg, for a quantity of second active ingredient equivalent to 100 mg of gabapentin or pregabalin.

Preferably, the pharmaceutical composition of the present invention comprises a quantity of first active ingredient equivalent to a quantity of trazodone between 0.250 and 0.100 mg, preferably between 0.200 and 0.111 mg, and more preferably between 0.167 and 0.125 mg, for a quantity of second active ingredient equivalent to 10 mg mirogabalin.

The compositions of gabapentin known in art generally comprise from 100 mg to 800 mg of gabapentin, particularly 100 mg, 300 mg, 400 mg, 600 mg or 800 mg of gabapentin. According to the invention, trazodone can be added to such compositions in order to provide a weight ratio, expressed as a weight ratio trazodone : gabapentin, between 1:40 and 1:100, preferably lower than 1:40 and up to 1:100, more preferably between 1:50 and 1:90, and even more preferably between 1:60 and 1:80 for combined use in the treatment of diabetic neuropathic pain.

According to a first embodiment, the pharmaceutical composition according to the present invention therefore comprises a quantity of second active ingredient equivalent to 100 mg of gabapentin and a quantity of first active ingredient equivalent to a quantity of trazodone between 2.50 and 1.00 mg, preferably from 2.00 to 1.11 mg, and more preferably from 1.67 to 1.25 mg.

According to a second embodiment, the pharmaceutical composition according to the present invention therefore comprises a quantity of second active ingredient equivalent to 300 mg of gabapentin and a quantity of first active ingredient equivalent to a quantity of trazodone between 7.50 and 3.00 mg, preferably from 6.00 to 3.33 mg, and more preferably from 5.00 to 3.75 mg.

According to a third embodiment, the pharmaceutical composition according to the present invention therefore comprises a quantity of second active ingredient equivalent to 400 mg of gabapentin and a quantity of first active ingredient equivalent to a quantity of trazodone between 10.00 and 4.00 mg, preferably 8.00 to 4.44 mg, and more preferably 6.67 to 5.00 mg.

Other embodiments of pharmaceutical composition according to the present invention may be considered within the scope of the invention as long as the weight ratio of trazodone : gabapentin is maintained.

The compositions of pregabalin known in the art generally comprise from 25 mg to 300 mg of pregabalin, in particular 25 mg, 50 mg, 75 mg, 100 mg, 150 mg, 200 mg, 225 mg and 300 mg of pregabalin. According to the invention, trazodone can be added to such compositions in order to provide a weight ratio, expressed as a weight ratio trazodone : pregabalin, between 1:40 and 1:100, preferably lower than 1:40 and up to 1:100, more preferably between 1:50 and 1:90, and even more preferably between 1:60 and 1:80 for combined use in the treatment of diabetic neuropathic pain.

According to a first embodiment, the pharmaceutical composition according to the present invention therefore comprises a quantity of second active ingredient equivalent to 50 mg of pregabalin and a quantity of first active ingredient equivalent to a quantity of trazodone between 1.25 and 0.50 mg, preferably from 1.00 to 0.56 mg, and more preferably from 0.83 to 0.63 mg.

According to a second embodiment, the pharmaceutical composition according to the present invention therefore comprises a quantity of second active ingredient equivalent to 100 mg of pregabalin and a quantity of first active ingredient equivalent to a quantity of trazodone between 2.50 and 1.00 mg, preferably from 2.00 to 1.11 mg, and more preferably from 1.67 to 1.25 mg.

According to a third embodiment, the pharmaceutical composition according to the present invention therefore comprises a quantity of second active ingredient equivalent to 300 mg of pregabalin and a quantity of first active ingredient equivalent to a quantity of trazodone between 7.50 and 3.00 mg, preferably from 6.00 to 3.33 mg, and more preferably from 5.00 to 3.75 mg.

Other embodiments of pharmaceutical composition according to the present invention may be considered within the scope of the invention as long as the weight ratio trazodone : pregabalin is maintained.

Mirogabalin compositions known in art generally comprise from 2.5 mg to 30 mg of mirogabalin, particularly 2.5 mg, 5 mg, 10 mg, 15 mg and 30 mg of mirogabalin. According to the invention, trazodone can be added to such compositions in order to provide a weight ratio, expressed as trazodone : mirogabalin, between 1:40 and 1 :100, preferably lower than 1:40 and up to 1:100, more preferably between 1:50 and 1:90, and even more preferably between 1:60 and 1:80 for combined use in the treatment of diabetic neuropathic pain.

According to a first embodiment, the pharmaceutical composition according to the present invention therefore comprises a quantity of second active ingredient equivalent to 5 mg of mirogabalin and a quantity of first active ingredient equivalent to a quantity of trazodone between 0.125 and 0.050 mg, preferably from 0.100 to 0.056 mg, and more preferably from 0.083 to 0.063 mg.

According to a second embodiment, the pharmaceutical composition according to the present invention therefore comprises a quantity of second active ingredient equivalent to 10 mg of mirogabalin and a quantity of first active ingredient equivalent to a quantity of trazodone between 0.250 and 0.100 mg, preferably 0.200 to 0.111 mg, and more preferably 0.067 to 0.125 mg.

According to a third embodiment, the pharmaceutical composition according to the present invention therefore comprises a quantity of second active ingredient equivalent to 15 mg of mirogabalin and a quantity of first active ingredient equivalent to a quantity of trazodone between 0.375 and 0.150 mg, preferably from 0.300 to 0.167 mg, and more preferably from 0.250 to 0.187 mg.

Other forms of pharmaceutical composition according to the present invention may be considered within the scope of the invention as long as the weight ratio trazodone : mirogabalin is maintained.

Gabapentin or pregabalin or mirogabalin prodrugs are included in the scope of the present invention. The chemically modified drug, or prodrug, should have a different pharmacokinetic profile from that of the parent, allowing easier absorption through the mucosal epithelium, improved salt formulation and/or solubility, improved systemic stability (e.g. for increased plasma half-life).

Such chemical modifications may be (i) ester derivatives, (ii) amide derivatives, (iii) carbamate derivatives, (iv) N-acyloxyalkyl derivatives, (v) N-acyloxyalkoxycarbonyl derivatives, (vi) peptides and (vii) any combination thereof.

The ester can be derived from the carboxylic group of the drug molecule by known means. The amide can be derived from the carboxylic or amine group of the drug molecule by known means. Derivatives of the ester or amide can be broken down, for example, from esterases or lipases.

The number of carbon atoms present in the compounds used to make these ester, amide, carbamate, N-acryloxyalkyl and N-acryloxyalkoxycarbonyl derivatives is equal to or lower than 10, preferably equal to or lower than 7, more preferably equal to or lower than 5. Useful examples of compounds are alcohols, carboxylic acids and amines having from 1 to 5 carbon atoms.

The peptide can be coupled to the drug molecule through the formation of amide bonds with the carboxylic or amine group of the drug molecule by known means. Peptides can be recognized by specific or non-specific proteinases.

The number of amino acids present in the compounds used to obtain these peptide derivatives is equal to or lower than 5, preferably equal to or lower than 4, more preferably equal to or lower than 3.

Carbamate, N-cyloxyalkoxyalkyl derivative and N-cyloxyalkoxycarbonyl derivative may be derived from the amino group of the drug molecule by known means. These derivatives may be broken down by esterases and/or decompose spontaneously.

Useful examples of suitable drugs are described, for example, in Stella V.J. et al., " Prodrug Strategies to overcome poor water solubility", Advance Drug Delivery Reviews 59 (2007) 677-694, and in Simplicio A.L. et al., "Prodrugs for Amines", Molecules 2008, 13, 519-547.

A particularly useful example of a gabapentin prodrug is gabapentin enacarbil, IUPAC chemical name 2-(1-{[({1-[(2-methylpropanoil)oxy]ethoxy}carbonyl) amino]methyl}cyclohexyl) acetic acid, represented by the following structural formula:

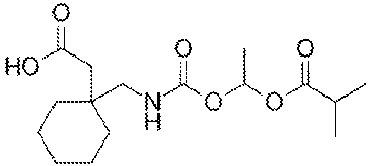

The salts of trazodone, gabapentin, pregabalin and mirogabalin are included in the scope of the present invention. The salt can be formed from physiologically acceptable organic and inorganic compounds having an acid or basic function.

Typical examples of suitable physiologically acceptable inorganic acids are hydrochloric acid, hydrobromic acid, sulphuric acid, phosphoric acid and nitric acid.

Typical examples of suitable physiologically acceptable organic acids are acetic acid, ascorbic acid, benzoic acid, citric acid, fumaric acid, lactic acid, maleic acid, methanesulfonic acid, oxalic acid, para-toluenesulfonic acid, benzenesulfonic acid, succinic acid, tannic acid and tartaric acid.

Typical examples of physiologically acceptable inorganic bases are ammonium, calcium, magnesium, sodium and potassium hydroxides, carbonates, and hydrogen carbonates, e.g. ammonium hydroxide, calcium hydroxide, magnesium carbonate, sodium carbonate and potassium hydrogen carbonate.

Typical examples of suitable physiologically acceptable organic bases are: arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, N-methylglucamine, glucamine, glucosamine, histidine, N-(2-hydroxyethyl)piperidine, N-(2-hydroxyethyl) pyrrolidine, isopropylamine, lysine, methylglucamine, morpholine, piperazine, piperidine, theobromine, triethylamine, trimethylamine, tripropylamine and trometamine.

Preferably, the pharmaceutical composition according to the present invention is for systemic use.

More preferably the pharmaceutical composition according to the present invention is formulated for oral or parenteral administration.

Preferably, the pharmaceutical composition according to the present invention is prepared in appropriate dosage forms.

Examples of appropriate dosage forms are: tablets, capsules, coated tablets, granules and solutions and syrups for oral administration; creams, ointments and antiseptic patches for topical administration; suppositories for rectal administration and sterile solutions for injection, aerosol or ophthalmic administration.

The preferred dosage forms are tablets, coated tablets, capsules and solutions for oral administration.

The dosage forms of the pharmaceutical composition of the present invention can be prepared according to techniques well known to pharmaceutical chemists, including mixing, granulation, compression, dissolution, sterilization and the like.

These dosage forms are advantageously formulated to ensure a controlled release of the active ingredient over time. In particular, depending on the type of therapy, the release time required can be very short, normal or long.

Preferably, the pharmaceutical composition of the present invention is contained in a single dosage form.

The present invention also comprises multiple dosage forms containing the quantities of the first and second active ingredient respectively to be taken simultaneously in order to administer said first and second active ingredient in a weight ratio between said first active ingredient and said second active ingredient, expressed as a weight ratio of trazodone : gabapentin or pregabalin or mirogabalin, between 1:40 and 1 :100, preferably lower than 1:40 and up to 1:100, preferably between 1:50 and 1:90, and even more preferably between 1:60 and 1:80.

For example, dosage forms containing 100 mg gabapentin or pregabalin may be combined with dosage forms containing from 2.50 to 1.00 mg, preferably from 2.00 to 1.11 mg, and more preferably from 1.67 to 1.25 mg trazodone for combined use in the treatment of diabetic neuropathic pain.

Similarly, dosage forms comprising 10 mg of mirogabalin may be associated with dosage forms comprising from 0.250 to 0.100 mg, preferably from 0.200 to 0.111 mg, and more preferably from 0.167 to 0.125 mg of trazodone for combined use in the treatment of diabetic neuropathic pain.

The pharmaceutically acceptable excipient can be selected from the group which comprises thickeners, gliders, binders, disintegrating agents, fillers, thinners, preservatives, stabilizers, surfactants, buffers, fluidifying agents, lubricants, wetting agents, absorbents, salts to regulate osmotic pressure, emulsifiers, flavorings, colorants, sweeteners and the like.

Particularly preferred excipients are sodium carbonate, magnesium carbonate, magnesium stearate, talc, sugars, lactose, pectin, dextrin, starch (especially corn starch), sodium starch glycolate, gelatin, microcrystalline cellulose, methylcellulose, ethylcellulose, sodium carboxymethylcellulose, povidone, cocoa butter, titanium dioxide (E171), red iron oxide and yellow iron oxide (E172), and the like.

The pharmaceutical composition according to the present invention has proven surprisingly useful in the treatment of pain resulting from diabetic neuropathy, but it is believed that it can also be used in the treatment of pain resulting from other types of neuropathies, particularly post-herpetic neuralgia, and post-surgical neuropathic pain.

The following examples are intended to further illustrate the present invention, but without limiting it.

### EXAMPLES

### EXAMPLE 1 - clinical study

The clinical study was carried out on a population of 142 patients with diabetic neuropathy of both sexes and aged between 18 and 75 years. Patients were divided into three groups and submitted to a randomized, double blind, placebo-controlled, parallel-group clinical trial, with double-dummy modality for patients in group 2.

Group 1 was treated with 20 mg trazodone (10 drops of 6% trazodone hydrochloride solution) three times a day for 8 weeks, for a total of 60 mg a day.

Group 2 was treated with 10 mg trazodone (5 drops of 6% trazodone hydrochloride solution) three times a day for 8 weeks, for a total of 30 mg a day.

Group 3 was treated with placebo, 10 drops of an oral solution three times a day for 8 weeks.

During the ninth week, concluding the clinical trial, group 1 was treated with 10 mg trazodone three times daily for a total of 30 mg per day, while both group 2 and group 3 were treated with placebo.

The 6% trazodone hydrochloride solution contained trazodone hydrochloride (6%), macrogol (35%), propylene glycol (30%), citric acid (0.5%), sucrose (0.15%), sodium hydroxide (0.125%), propyl gallate (0.10%), disodium edetate (0.05%), and purified water as needed at 100 mL. The placebo solution is identical to trazodone solution, but without active ingredient.

Gabapentin was administered in an incremental dosage for all patients treated according to the dosage regimen shown in Table 1 below using capsules containing 100 mg, 300 mg and 400 mg of commercially available gabapentin (Neurontin^{®}, Pfizer). The capsules contain lactose monohydrate, corn starch and talc as excipients, and E171 and E772 color combinations.

**TABLE 1**

| Capsule content (mg) | Posology | Period | Dosage (mg/day) |
|---|---|---|---|
| 100 | 1 capsule 3 times a day | 1 ^{st} week | 300 |
| 100 | 2 capsules 3 times a day | 2^{nd} week | 600 |
| 300 | 1 capsule 3 times a day | 3^{rd} week | 900 |
| 400 | 1 capsule 3 times a day | 4^{th} week | 1200 |
| 300 | 2 capsules 3 times a day | 5^{th} week | 1800 |
| 400 | 2 capsules 3 times a day | 6^{th}-8^{th} week | 2400 |

Table 2 below shows the trazodone : gabapentin ratio for each group 1 and 2 during treatment:

**TABLE 2**

| | Group 1 | Group 2 |
|---|---|---|
| 1^{st} week | 1:5 | 1:10 |
| 2^{nd} week | 1:10 | 1:20 |
| 3^{rd} week | 1:15 | 1:30 |
| 4^{th} week | 1:20 | 1:40 |
| 5^{th} week | 1:30 | 1:60 |
| 6^{th} week | 1:40 | 1:80 |
| 7^{th} week | 1:40 | 1:80 |
| 8^{th} week | 1:40 | 1:80 |
| 9^{th} week | 1:80 | n.a. |

At the beginning of treatment and at the end of each treatment week, the patients under treatment completed the Brief Pain Inventory - Short Form (BPI-SF), which includes a set of questions relating to pain self-assessment (Zelman DC, et al., "Validation of a modified version of the pain inventory brief for painful diabetic peripheral neuropathy", J. Pain Symptom Manage. 2005 Apr; 29(4):401-10).

In particular, patients assessed the average pain intensity in the week prior to the assessment (issue 5) and at the time of the assessment (issue 6) on a scale from 0 to 10 where 0 represents "no pain" and 10 represents "more pain than you can imagine".

At each visit the evaluations were collected and processed in order to determine the deviation from the average value obtained for each group at the beginning of the treatment. The results were collected in the following Tables 3 and 4, from which the graphs shown in Figures 1 and 2 were obtained, respectively.

**TABLE 3**

| Question 5 | Average pain assessment deviation from beginning of treatment (Visit 0) | | |
|---|---|---|---|
| | Group 1 | Group 2 | Group 3 |
| Visit 1 | -0.7 | -1.1 | -1.0 |
| Visit 2 | -1.4 | -1.6 | -1.7 |
| Visit 3 | -1.7 | -2.0 | -2.1 |
| Visit 4 | -2.1 | -2.8 * | -1.9 |
| Visit 5 | -2.2 | -2.9 ** | -2.1 |
| Visit 6 | -2.3 | -2.8 *** | -2.4 |
| Visit 7 | -2.6 | -3.0 *** | -2.4 |
| Visit 8 | -2.6 | -3.1 *** | -2.5 |
| Visit 9 | -2.7 | -3.2 ** | -2.5 |

| | | | |
|---|---|---|---|
| * p<0.05 vs group 3 ** p<0.1 vs group 3 *** p<0.5 vs group 3 | | | |

**TABLE 4**

| Question 6 | Average pain assessment deviation from beginning of treatment (Visit 0) | | |
|---|---|---|---|
| | Group 1 | Group 2 | Group 3 |
| Visit 1 | -0.6 | -1.0 | -0.9 |
| Visit 2 | -1.4 | -1.4 | -1.4 |
| Visit 3 | -1.6 | -1.9 | -1.7 |
| Visit 4 | -2.2 | -2.5 * | -1.6 |
| Visit 5 | -2.2 | -2.6 ** | -1.8 |
| Visit 6 | -2.2 | -2.7 ** | -2.0 |
| Visit 7 | -2.6 | -2.7 ** | -1.9 |
| Visit 8 | -2.4 | -2.9 * | -2.0 |
| Visit 9 | -2.6 | -3.1 * | -2.0 |

| | | | |
|---|---|---|---|
| * p<0.05 vs group 3 ** p<0.1 vs group 3 *** p<0.5 vs group 3 | | | |

Figure 1 describes the deviation from the initial pain intensity value in the week preceding the assessment (question 5). At the end of the 4^{th} and 5^{th} week, group 2 showed a significant reduction in pain by almost one point compared to group 1 and 3, which then remained at about half a point in the following weeks.

Figure 2 describes the deviation from the initial pain intensity value at the time of assessment (question 6). At the end of the 4^{th} week and throughout the subsequent period until the end of treatment, group 2 showed a significant reduction in pain by almost one point compared to group 3 and about half a point compared to group 1.

The effect obtained with group 2 patients is all the more surprising considering that the amount of trazodone administered to group 2 is half the amount administered to group 1, to the extent that a synergistic effect linked to the relative amounts of trazodone and gabapentin for ratios lower than 1:40 is plausible.

The effect obtained with group 2 patients also shows a linear and progressively decreasing trend in the last weeks of treatment, thus giving the feeling that the prolongation of the treatment could have led to further reductions in the perception of pain, unlike the trend resulting from group 1 and 3 patients where the curve seems to show a constant trend, with a flattening after the sixth week.

Finally, Table 5 below shows for each patient group and for the answers given to question 5 (i) the sum of all the deviations obtained at each visit, (ii) the percentage of patients with a reduction of at least 50% compared to the initial value, and (iii) the relative NNT index (Number Needed to Treat).

**TABLE 5**

| | Group 1 | Group 2 | Group 3 |
|---|---|---|---|
| Sum deviations | -116.3 | -142.5 ** | -117.6 |
| Patients with 50% reduction | 54.0 | 62.8 | 45.8 |
| NNT | 12.2 | 5.9 | - |

| | | | |
|---|---|---|---|
| ** p<0.5 vs group 3 | | | |

These results confirmed the surprising effect of group 2 treatment, with a significant increase in overall pain reduction compared to both group 1 and group 3, and an extremely favorable and promising NNT value.

The patients under treatment also completed the SF-36 Health Status Assessment Questionnaire (G. Apolone et al., " Questionario sullo stato di salute SF-36. Manuale d'uso e guida all'interpretazione dei risultati", Editore Guerini e Associati, Nona Edizione, Gennaio 2005).

The SF-36 questionnaire comprises a set of 36 questions defining eight different aspects of general wellness, namely physical activity (PF - physical functioning), role and physical health (RP - role participation), physical pain (BP - bodily pain), general health (GH - general health), vitality (VT - vitality), social activities (SF - social functioning), role and emotional state (RE - emotional role), and mental health (MH - mental health).

The information obtained from these eight aspects has been aggregated to define the Physical Component Summary (PCS) and Mental Component Summary (MCS) of health status.

MCS is mainly related to aspects of vitality (VT), social activities (SF), role and emotional state (RE) and mental health (MH) and is a measure used to estimate the percentage of patients with a positive risk of depression or anxiety compared to the normal general population.

The results have been collected in Table 6 below, from which the graph shown in Figure 3 has been obtained.

The results are expressed as a percentage of patients with a positive risk of depression or anxiety compared to the normal general population in which a normal value of 18 is estimated.

**TABLE 6**

| | Group 1 | Group 2 | Group 3 |
|---|---|---|---|
| Visit 0 | 31 | 52 | 38 |
| Visit 8 | 25 | 21 | 29 |

A marked effect of group 2 treatment has been shown to reduce the risk of depression in patients with diabetic neuropathy.

Although at the beginning of treatment (Visit 0) a higher percentage (52%) of patients in group 2 had a positive risk of depression compared to group 1 (31%) and group 3 (38%), at the end of treatment (Visit 8) the risk percentage in group 2 was reduced to 21% (compared to 25% in group 1 and 29% in group 3). This value is very close to the estimated risk of depression in the general population (indirect control) of 18%.

The results of Table 6 for Group 2 further confirm the hypothesis of a synergistic effect linked to the relative amounts of trazodone and gabapentin for ratios lower than 1:40.

### EXAMPLE 2 - formulations

The following Tables 7 and 8 provide some non-exhaustive examples of trazodone and gabapentin formulations for use according to the present invention:

**TABLE 7**

| Capsules | mg | mg | mg |
|---|---|---|---|
| Gabapentin | 100 | 300 | 400 |
| Trazodone | 1.25 | 3.75 | 5.00 |
| Lactose | 13 | 41 | 54 |
| Corn starch | 40 | 40 | 40 |
| Talc | 5 | 5 | 5 |

**TABLE 8**

| Tablets | mg | mg | mg |
|---|---|---|---|
| Gabapentin | 100 | 300 | 400 |
| Trazodone | 1.50 | 4.50 | 6.00 |
| Lactose | 200 | 600 | 600 |
| Starch | 50 | 50 | 50 |
| Sodium starch glycolate | 15 | 45 | 45 |
| Povidone | 5 | 5 | 5 |
| Magnesium stearate | 4 | 4 | 4 |

## Claims

1. A pharmaceutical composition which comprises (a) a combination of (i) a first active ingredient selected from the group consisting of trazodone and a salt thereof, and (ii) a second active ingredient selected from the group consisting of gabapentin, pregabalin, mirogabalin, a salt thereof, and a prodrug thereof, wherein said prodrug is gabapentin enacarbil, and (b) at least one pharmaceutically acceptable excipient, for use in the treatment of pain resulting from diabetic neuropathy, wherein said pharmaceutical composition comprises a weight ratio of said first active ingredient to said second active ingredient, expressed as a weight ratio of trazodone : gabapentin or pregabalin or mirogabalin lower than 1:40 and up to 1:100.

2. The pharmaceutical composition for use according to claim 1, wherein said pharmaceutical composition comprises a weight ratio of said first active ingredient and said second active ingredient, expressed as the weight ratio of trazodone : gabapentin or pregabalin or mirogabalin, between 1:50 and 1:90.

3. The pharmaceutical composition for use according to claim 1, wherein said pharmaceutical composition comprises a weight ratio of said first active ingredient to said second active ingredient, expressed as the weight ratio of trazodone : gabapentin or pregabalin or mirogabalin, between 1:60 and 1:80.

4. The pharmaceutical composition for use according to claim 1, wherein said pharmaceutical composition comprises a quantity of first active ingredient equivalent to a quantity of trazodone between 2.50 and 1.00 mg, preferably from 2.00 to 1.11 mg, and more preferably from 1.67 to 1.25 mg, for a quantity of second active ingredient equivalent to 100 mg of gabapentin or pregabalin.

5. The pharmaceutical composition for use according to claim 1, wherein said pharmaceutical composition comprises a quantity of second active ingredient equivalent to 100 mg of gabapentin and a quantity of first active ingredient equivalent to a quantity of trazodone between 2.50 and 1.00 mg, preferably 2.00 to 1.11 mg, and more preferably 1.67 to 1.25 mg.

6. The pharmaceutical composition for use according to claim 1, wherein said pharmaceutical composition comprises a quantity of second active ingredient equivalent to 300 mg of gabapentin and a quantity of first active ingredient equivalent to a quantity of trazodone between 7.50 and 3.00 mg, preferably from 6.00 to 3.33 mg, and more preferably from 5.00 to 3.75 mg.

7. The pharmaceutical composition for use according to claim 1, wherein said pharmaceutical composition comprises a quantity of second active ingredient equivalent to 400 mg of gabapentin and a quantity of first active ingredient equivalent to a quantity of trazodone between 10.00 and 4.00 mg, preferably 8.00 to 4.44 mg, and more preferably 6.67 to 5.00 mg.

8. The pharmaceutical composition for use according to claim 1, wherein said pharmaceutical composition comprises a quantity of second active ingredient equivalent to 50 mg of pregabalin and a quantity of first active ingredient equivalent to a quantity of trazodone between 1.25 and 0.50 mg, preferably 1.00 to 0.56 mg, and more preferably 0.83 to 0.63 mg.

9. The pharmaceutical composition for use according to claim 1, wherein said pharmaceutical composition comprises a quantity of second active ingredient equivalent to 100 mg of pregabalin and a quantity of first active ingredient equivalent to a quantity of trazodone between 2.50 and 1.00 mg, preferably 2.00 to 1.11 mg, and more preferably 1.67 to 1.25 mg.

10. The pharmaceutical composition for use according to claim 1, wherein said pharmaceutical composition comprises a quantity of second active ingredient equivalent to 300 mg of pregabalin and a quantity of first active ingredient equivalent to a quantity of trazodone between 7.50 and 3.00 mg, preferably 6.00 to 3.33 mg, and more preferably 5.00 to 3.75 mg.

11. The pharmaceutical composition for use according to claim 1, wherein said pharmaceutical composition comprises a quantity of first active ingredient equivalent to a quantity of trazodone between 0.250 and 0.100 mg, preferably between 0.200 and 0.111 mg, and more preferably between 0.167 and 0.125, for a quantity of second active ingredient equivalent to 10 mg mirogabalin.

12. The pharmaceutical composition for use according to any one of the preceding claims wherein said salt is formed with physiologically acceptable organic and inorganic acids or bases.

13. The pharmaceutical composition for use according to any one of the preceding claims wherein said first active ingredient and said second active ingredient are incorporated into a single dosage form or are incorporated into a first dosage form containing said first active ingredient and a second dosage form containing said second active ingredient, respectively.

14. The pharmaceutical composition for use according to claim 13 wherein said dosage form is selected from the group consisting of tablets, coated tablets, capsules and solutions for oral administration.

15. The pharmaceutical composition as defined in any one of the preceding claims for use in the treatment of pain resulting from post-herpetic neuralgia and post-surgical neuropathic pain.

## Patentansprüche

1. Pharmazeutische Zusammensetzung, umfassend (a) eine Kombination aus (i) einem ersten Wirkstoff, ausgewählt aus der Gruppe, bestehend aus Trazodon und einem Salz davon, und (ii) einem zweiten Wirkstoff, ausgewählt aus der Gruppe, bestehend aus Gabapentin, Pregabalin, Mirogabalin, einem Salz davon, und einem Prodrug davon, wobei der Prodrug Gabapentin enacarbil ist, und (b) mindestens einen pharmazeutisch annehmbaren Hilfsstoff zur Verwendung bei der Behandlung von Schmerzen, die aus diabetischer Neuropathie resultieren, wobei die pharmazeutische Zusammensetzung ein Gewichtsverhältnis des ersten Wirkstoffs zu dem zweiten Wirkstoff, ausgedrückt als ein Gewichtsverhältnis von Trazodon : Gabapentin oder Pregabalin oder Mirogabalin von weniger als 1:40 und bis zu 1:100 umfasst.

2. Pharmazeutische Zusammensetzung zur Verwendung nach Anspruch 1, wobei die pharmazeutische Zusammensetzung ein Gewichtsverhältnis des ersten Wirkstoffs und des zweiten Wirkstoffs, ausgedrückt als das Gewichtsverhältnis von Trazodon : Gabapentin oder Pregabalin oder Mirogabalin, zwischen 1:50 und 1:90 umfasst.

3. Pharmazeutische Zusammensetzung zur Verwendung nach Anspruch 1, wobei die pharmazeutische Zusammensetzung ein Gewichtsverhältnis des ersten Wirkstoffs zu dem zweiten Wirkstoff, ausgedrückt als das Gewichtsverhältnis von Trazodon : Gabapentin oder Pregabalin oder Mirogabalin, zwischen 1:60 und 1:80 umfasst.

4. Pharmazeutische Zusammensetzung zur Verwendung nach Anspruch 1, wobei die pharmazeutische Zusammensetzung eine Menge eines ersten Wirkstoffs umfasst, die einer Menge an Trazodon zwischen 2,50 und 1,00 mg, bevorzugt von 2,00 bis 1,11 mg und mehr bevorzugt von 1,67 bis 1,25 mg entspricht, bei einer Menge des zweiten Wirkstoffs, die 100 mg Gabapentin oder Pregabalin entspricht.

5. Pharmazeutische Zusammensetzung zur Verwendung nach Anspruch 1, wobei die pharmazeutische Zusammensetzung eine Menge eines zweiten Wirkstoffs umfasst, die 100 mg Gabapentin entspricht, und eine Menge eines ersten Wirkstoffs, die einer Menge an Trazodon zwischen 2,50 und 1,00 mg, bevorzugt 2,00 bis 1,11 mg und mehr bevorzugt 1,67 bis 1,25 mg entspricht.

6. Pharmazeutische Zusammensetzung zur Verwendung nach Anspruch 1, wobei die pharmazeutische Zusammensetzung eine Menge eines zweiten Wirkstoffs umfasst, die 300 mg Gabapentin entspricht, und eine Menge eines ersten Wirkstoffs, die einer Menge an Trazodon zwischen 7,50 und 3,00 mg, bevorzugt von 6,00 und 3,33 mg und mehr bevorzugt von 5,00 und 3,75 mg entspricht.

7. Pharmazeutische Zusammensetzung zur Verwendung nach Anspruch 1, wobei die pharmazeutische Zusammensetzung eine Menge eines zweiten Wirkstoffs umfasst, die 400 mg Gabapentin entspricht, und eine Menge eines ersten Wirkstoffs, die einer Menge an Trazodon zwischen 10,00 und 4,00 mg, bevorzugt 8,00 bis 4,44 mg und mehr bevorzugt 6,67 bis 5,00 mg entspricht.

8. Pharmazeutische Zusammensetzung zur Verwendung nach Anspruch 1, wobei die pharmazeutische Zusammensetzung eine Menge eines zweiten Wirkstoffs umfasst, die 50 mg Pregabalin entspricht, und eine Menge eines ersten Wirkstoffs, die einer Menge an Trazodon zwischen 1,25 und 0,50 mg, bevorzugt 1,00 bis 0,56 mg und mehr bevorzugt 0,83 bis 0,63 mg entspricht.

9. Pharmazeutische Zusammensetzung zur Verwendung nach Anspruch 1, wobei die pharmazeutische Zusammensetzung eine Menge eines zweiten Wirkstoffs umfasst, die 100 mg Pregabalin entspricht, und eine Menge eines ersten Wirkstoffs, die einer Menge an Trazodon zwischen 2,50 und 1,00 mg, bevorzugt 2,00 bis 1,11 mg und mehr bevorzugt 1,67 bis 1,25 mg entspricht.

10. Pharmazeutische Zusammensetzung zur Verwendung nach Anspruch 1, wobei die pharmazeutische Zusammensetzung eine Menge eines zweiten Wirkstoffs umfasst, die 300 mg Pregabalin entspricht, und eine Menge eines ersten Wirkstoffs, die einer Menge an Trazodon zwischen 7,50 und 3,00 mg, bevorzugt 6,00 bis 3,33 mg und mehr bevorzugt 5,00 bis 3,75 mg entspricht.

11. Pharmazeutische Zusammensetzung zur Verwendung nach Anspruch 1, wobei die pharmazeutische Zusammensetzung eine Menge eines ersten Wirkstoffs umfasst, die einer Menge an Trazodon zwischen 0,250 und 0,100 mg, bevorzugt zwischen 0,200 und 0,111 mg und mehr bevorzugt zwischen 0,167 und 0,125 mg entspricht, für eine Menge eines zweiten Wirkstoffs, die 10 mg Mirogabalin entspricht.

12. Pharmazeutische Zusammensetzung zur Verwendung nach einem beliebigen der vorhergehenden Ansprüche, wobei das Salz mit physiologisch akzeptablen organischen und anorganischen Säuren oder Basen gebildet ist.

13. Pharmazeutische Zusammensetzung zur Verwendung nach einem beliebigen der vorhergehenden Ansprüche, wobei der erste Wirkstoff und der zweite Wirkstoff in eine einzige Dosierungsform eingearbeitet sind oder in eine erste Dosierungsform, die den ersten Wirkstoff enthält, und eine zweite Dosierungsform, die den zweiten Wirkstoff enthält, eingearbeitet sind.

14. Pharmazeutische Zusammensetzung zur Verwendung nach Anspruch 13, wobei die Dosierungsform ausgewählt ist aus der Gruppe bestehend aus Tabletten, überzogenen Tabletten, Kapseln und Lösungen zur oralen Verabreichung.

15. Pharmazeutische Zusammensetzung, wie in einem beliebigen der vorhergehenden Ansprüche definiert, zur Verwendung bei der Behandlung von Schmerzen, die aus postherpetischer Neuralgie und postoperativen neuropathischen Schmerzen resultieren.

## Revendications

1. Composition pharmaceutique qui comprend (a) une combinaison de (i) un premier ingrédient actif sélectionné dans le groupe constitué de la trazodone et d'un sel de celle-ci, et (ii) un second ingrédient actif sélectionné dans le groupe constitué de la gabapentine, prégabaline, mirogabaline, un sel de celles-ci, et un promédicament de celles-ci, ledit promédicament étant le gabapentine énacarbil, et (b) au moins un excipient pharmaceutiquement acceptable, destiné à une utilisation dans le traitement de la douleur résultant de la neuropathie diabétique, ladite composition pharmaceutique comprenant un rapport en poids dudit premier ingrédient actif audit second ingrédient actif, exprimé comme un rapport en poids de trazodone:gabapentine ou prégabaline ou mirogabaline inférieur à 1:40 et jusqu'à 1:100.

2. Composition pharmaceutique destinée à une utilisation selon la revendication 1, ladite composition pharmaceutique comprenant un rapport en poids dudit premier ingrédient actif et dudit second ingrédient actif, exprimé comme le rapport en poids de trazodone:gabapentine ou prégabaline ou mirogabaline, entre 1:50 et 1:90.

3. Composition pharmaceutique destinée à une utilisation selon la revendication 1, ladite composition pharmaceutique comprenant un rapport en poids dudit premier ingrédient actif audit second ingrédient actif, exprimé comme le rapport en poids de trazodone:gabapentine ou prégabaline ou mirogabaline, entre 1:60 et 1:80.

4. Composition pharmaceutique destinée à une utilisation selon la revendication 1, ladite composition pharmaceutique comprenant une quantité de premier ingrédient actif équivalente à une quantité de trazodone entre 2,50 et 1,00 mg, préférablement de 2,00 à 1,11 mg, et plus préférablement de 1,67 à 1,25 mg, pour une quantité de second ingrédient actif équivalente à 100 mg de gabapentine ou de prégabaline.

5. Composition pharmaceutique destinée à une utilisation selon la revendication 1, ladite composition pharmaceutique comprenant une quantité de second ingrédient actif équivalente à 100 mg de gabapentine et une quantité de premier ingrédient actif équivalente à une quantité de trazodone entre 2,50 et 1,00 mg, préférablement de 2,00 à 1,11 mg, et plus préférablement de 1,67 à 1,25 mg.

6. Composition pharmaceutique destinée à une utilisation selon la revendication 1, ladite composition pharmaceutique comprenant une quantité de second ingrédient actif équivalente à 300 mg de gabapentine et une quantité de premier ingrédient actif équivalente à une quantité de trazodone entre 7,50 et 3,00 mg, préférablement de 6,00 à 3,33 mg, et plus préférablement de 5,00 à 3,75 mg.

7. Composition pharmaceutique destinée à une utilisation selon la revendication 1, ladite composition pharmaceutique comprenant une quantité de second ingrédient actif équivalente à 400 mg de gabapentine et une quantité de premier ingrédient actif équivalente à une quantité de trazodone entre 10,00 et 4,00 mg, préférablement de 8,00 à 4,44 mg, et plus préférablement 6,67 à 5,00 mg.

8. Composition pharmaceutique destinée à une utilisation selon la revendication 1, ladite composition pharmaceutique comprenant une quantité de second ingrédient actif équivalente à 50 mg de prégabaline et une quantité de premier ingrédient actif équivalente à une quantité de trazodone entre 1,25 et 0,50 mg, préférablement de 1,00 à 0,56 mg, et plus préférablement de 0,83 à 0,63 mg.

9. Composition pharmaceutique destinée à une utilisation selon la revendication 1, ladite composition pharmaceutique comprenant une quantité de second ingrédient actif équivalente à 100 mg de prégabaline et une quantité de premier ingrédient actif équivalente à une quantité de trazodone entre 2,50 et 1,00 mg, préférablement de 2,00 à 1,11 mg, et plus préférablement de 1,67 à 1,25 mg.

10. Composition pharmaceutique destinée à une utilisation selon la revendication 1, ladite composition pharmaceutique comprenant une quantité de second ingrédient actif équivalente à 300 mg de prégabaline et une quantité de premier ingrédient actif équivalente à une quantité de trazodone entre 7,50 et 3,00 mg, préférablement de 6,00 à 3,33 mg, et plus préférablement de 5,00 à 3,75 mg.

11. Composition pharmaceutique destinée à une utilisation selon la revendication 1, ladite composition pharmaceutique comprenant une quantité de premier ingrédient actif équivalente à une quantité de trazodone entre 0,250 et 0,100 mg, préférablement entre 0,200 et 0,111 mg, et plus préférablement entre 0,167 et 0,125, pour une quantité de second ingrédient actif équivalente à 10 mg de mirogabaline.

12. Composition pharmaceutique destinée à une utilisation selon l'une quelconque des revendications précédentes, ledit sel étant formé avec des acides ou des bases organiques et inorganiques physiologiquement acceptables.

13. Composition pharmaceutique destinée à une utilisation selon l'une quelconque des revendications précédentes, ledit premier ingrédient actif et ledit second ingrédient actif étant incorporés en une forme galénique unique ou étant incorporés en une première forme galénique contenant ledit premier ingrédient actif et une seconde forme galénique contenant ledit second ingrédient actif, respectivement.

14. Composition pharmaceutique destinée à une utilisation selon la revendication 13, ladite forme galénique étant sélectionnée dans le groupe constitué des comprimés, des comprimés enrobés, des capsules et des solutions pour l'administration par voie orale.

15. Composition pharmaceutique telle que définie selon l'une quelconque des revendications précédentes destinée à une utilisation dans le traitement d'une douleur résultant d'une névralgie post-herpétique et d'une douleur neuropathique post-opératoire.
